# EUROPEAN PATENT APPLICATION

(11) **EP 0 592 381 A1**
(43) Date of publication of application: **13.04.1994**
(21) Application number: 93850187.1
(22) Date of filing: 05.10.1993
(51) Int. Cl.: A61M 16/00

(54) **Anaesthesia apparatus**

(30) Priority: 07.10.1992 SE 9202947
(71) Applicant: Mentell, Ove, S-724 79 Västeras (SE)
(72) Inventor: Mentell, Ove, S-724 79 Västeras (SE)
(74) Representative: Mossmark, Anders

(57) **Abstract**

Anaesthesia apparatus comprising means (2, 3) for connection to a patient, to a gas source (40) for narcosis gas and oxygen gas, to a unit (6) for control of the respiratory process and to a ventilator (7) for respiratory assistance as well as to an absorber (18) for carbon dioxide. A conduit (1) supports connection means (2, 3) at an outer end and, at its inner end (4), is connected to the respiratory control (6), to the ventilator (7) and to the absorber (18). A conduit (35) from the gas source (40) has its orifice close to the patient connection (2, 3). The first said conduit (1) is arranged, between its connections, to form a reservoir for gas exhaled by the patient which is adapted in terms of size to give the patient a contribution of exhaled gas quantified with carbon dioxide during respiration such that the risk of hypocapnia due to a relatively too large contribution of oxygen can be controlled. The gas for which there is no room in the reservoir passes through the absorber (18) and returns to the patient whereby the content of the narcosis gas and oxygen gas in this surplus gas can be used beneficially.

## Description

### TECHNICAL FIELD:

The present invention relates to an anaesthesia apparatus of the type defined in the preamble of claim 1.

### STATE OF THE ART:

The apparatus used in modern medical treatment for administering narcosis to a patient basically comprises two different main types. With the first main type, which works according to the so-called Bain-system, fresh gas and thus oxygen is supplied for the patient's metabolism as well as anaesthesia gas for the narcosis, via a separate conduit which is connected close to the connection of the apparatus to the patient. Additionally a conduit having a certain volume is connected to the patient, said conduit receiving exhaled air during exhalation, said exhaled air in addition to the elements of the supplied gas comprising carbon dioxide and water vapour produced by the patient. During inhalation a certain part of the exhaled air is sucked back from the latter conduit together with the fresh gas supplied via the particular conduit. Since fresh gas is supplied, an increase in volume occurs in the gas circulated via the patient, which surplus is released through a discharge valve. Means can also be connected to the system for controlling and assisting the patient's respiration; a so-called ventilator. One of the important characteristics of the Bain-system is that the particular conduit for the fresh gas is situated coaxially inside said conduit which takes up the exhaled gas and which constitutes a reservoir for re-inhaled gas. This arrangement makes the system of tubes and connection members easily manageable.

With the second main type of anaesthesia apparatus which works according to what is commonly called the circle system, the carbon dioxide supplied from the patient is removed in an absorber, through which the exhaled air is allowed to circulate. By the use of one-way valves the exhaled air is directed into the absorber and the gas, purified from carbon dioxide, is directed through a second valve in a direction towards the connection location to the patient for re-inhalation. Somewhere in this circulation system fresh gas is added as a replacement for the gas which is taken-up by the patient. A ventilator can also be used with the circle system for producing rhythmic pressure changes to assist or replace the patient's spontaneous respiration.

It will be clear that the Bain-system is significantly simpler in its construction than the circle system, particularly due to fact that there is no absorber and thus no consumption of CO₂-absorbing material either. The inhaled gas comprises a certain amount of carbon dioxide and also moisture. In this way the risk of hypocapnia is reduced or eliminated due to the fact that a part of the inhaled gas consists of exhaled gas which thus comprises carbon dioxide. At the same time a natural recirculation of the moisture from the respiratory passages is obtained, which contributes to the mucous membranes not drying out.

The circle system is thus more complicated in its construction with double tubes at the patient connection and with directional valves for the gas in the absorber's circle system. The supplied gas lacks said carbon dioxide component to a higher degree than with the Bain-system. This results in a risk of hypocapnia or hyperventilation, an undesirable condition which occurs even in connection with normal respiratory volumes and frequencies. The carbon dioxide absorber however helps to warm and moisturize the gas.

The aforesaid advantages of the Bain-system meant that it received a great deal of attention and also widespread use during a certain period. The consumption of fresh gas was however significantly larger when using the Bain-system than when using the circle system with which exhaled air is regenerated and which can constitute the inhaled gas to a large degree and whereby the need for fresh gas is reduced. Since consequently less fresh gas is supplied than with the Bain-system the release of surplus gas is also less. Surplus gas comprises anaesthesia gas not taken-up by the patient and this has to be dealt with in some way in a ventilation system and generally ends up in the air of the surroundings. With increased supply of gas there is also a risk for increased release into the locality where the patient is sleeping. Thus, when using the Bain-system rather than the circle system, an unfavourable effect on the environment of the surroundings as well as the working environment occurs, added to which are also the increased costs of gas consumption.

Due to a greater awareness about the environment and of costs this has meant in more and more cases that the Bain-system has been given up in favour of the circle system and also to the acceptance that more attention must be given to the risk of hypocapnia.

### SUMMARY OF THE INVENTION:

The apparatus according to the invention can be characterized as representing a hybrid system whereby the advantages of the circle system with more limited consumption of gas are combined with the capacity of the Bain-system to give a certain self-regulation of the character of the inhaled gas in a form more suitable for the patient. The regulation itself gives the effect that this character of the inhaled air is maintained for a wide range of patients with differing physique.

One advantage with the apparatus according to the invention is thus that the adjustment of the apparatus for adaptation of the same to various patients is simplified.

Said advantages are achieved by forming the apparatus with the features defined in claim 1.

### DESCRIPTION OF THE FIGURES:

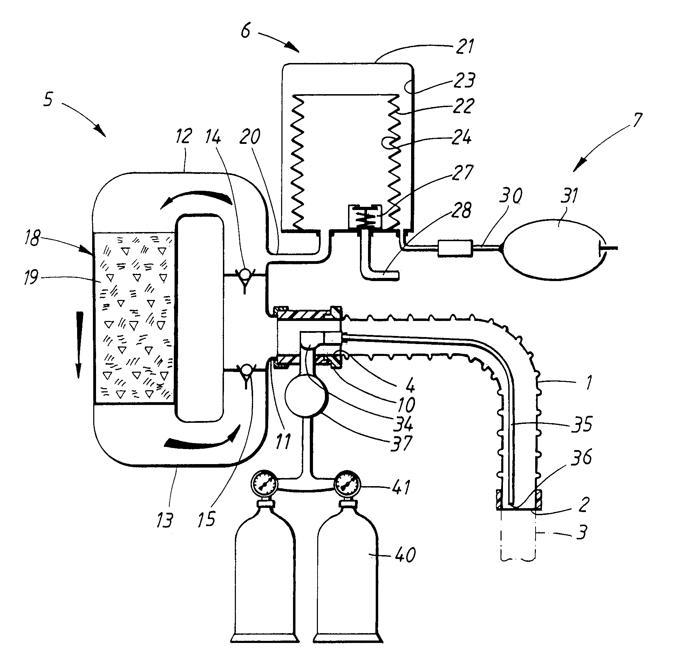

An embodiment of the invention will now be described as illustrated in the accompanying drawing depicting a schematic representation of the apparatus according to the invention.

### DESCRIPTION OF PREFERRED EMBODIMENTS:

In the anaesthesia apparatus according to the invention which is shown schematically in the drawing, reference numeral 1 denotes a connection tube with an outer end 2 to which the patient's respiratory organs are attached preferably by means of an oropharyngeal tube 3 indicated in dashed lines. The tube 1 is connected with its inner end 4 to the apparatus part of an anaesthesia apparatus which comprises a carbon dioxide absorber 5 having connection conduits and one-way valves, a bellows unit 6 with connection conduits and valve equipment as well as a ventilator 7 for producing artificial respiration for the patient when this is required.

The inner end 4 of the tube 1 is fixed to a connection piece 10 which continues into a pipe 11 with two branches 12 and 13. In the branch 12 there is a one-way valve 14 which allows flow in one direction only (shown with an arrow in branch 12). The flow can thereby occur in the direction from the connection piece 10. A one-way valve 15 which allows flow in one direction only (shown with an arrow in branch 13) is placed in the branch 13. This flow direction is out towards the connection piece 10.

A carbon dioxide absorber 18 is placed between the branches and comprises an exchangeable cartridge with an absorption material 19 for carbon dioxide. Due to the function of the one-way valves 14 and 15, the direction of flow through the absorber is as shown with an arrow.

The said bellows unit is connected to the branch 12 by means of a conduit 20. The bellows unit itself consists of a hard outer cover 29 of transparent material together with a bellows 22 of soft foldable material such as rubber arranged inside said cover. Due to this arrangement, an outer chamber 23 is formed between the outer side of the bellows 22 and the inner wall of the cover and inside the bellows an inner chamber 24 is formed with both chambers being separated from one another by means of the bellows wall. The conduit 20 is connected inside the inner chamber 24. An over-pressure valve 27 is also connected to the same, said over-pressure valve allowing gas from the inner chamber 24 to flow out at a certain pressure by means of an outlet conduit 28 which is connected to some type of ventilation system.

The ventilator 7 for its part is connected to the outer chamber 23 in the bellows unit by means of a conduit 30. The active part of the bellows unit is shown here as a soft bladder 31 by means of which manual ventilation can occur upon rhythmic squashing of the bladder. The anaesthesia apparatus can, and is, suitably also connected to a ventilator machine which can be operated to give a rhythmical pressure variation with adjustable frequency. Hand ventilators as well as ventilator machines are well known in the art and therefore do not need to be described in further detail here. See for example US-A-3 307 542.

A nipple 34 is positioned in the connection piece 10, to which nipple a thin tube 35 is connected, said tube being positioned in the larger tube 1. The tube 35 has its orifice at an open end 36 at the outer end 2 of the tube 1. Sources of narcosis gas and oxygen are connected to the nipple via a regulation device 37, said sources being represented by two gas cylinders 40 with connecting pipes as well as valve and pressure measurement means 41. The gas source is often a central system, to which connection occurs by means of fixedly arranged pipes.

The regulation device 37 allows control of the quantities and proportions of the oxygen and narcosis gases which flow through the tube 35 via the nipple 34 and out to its open end 36. Measuring devices are suitably arranged for controlling the flow of gas.

When using the anaesthesia apparatus, the outer end 2 of the tube 1 is connected to the patient preferably by means of the oropharyngeal tube 3. A mixture of narcosis gas and oxygen gas is supplied from the gas source 40 via the regulation device 37 and the nipple 34 and through the tube 35, from which the gas flows out through the open end 36. The outlet flow thus occurs in close proximity to the patient's respiratory passages and consequently the gas is supplied in a very direct manner without first having passed any gas-filled channels or chambers within the anaesthesia apparatus.

The supplied gas which reaches the lungs' alveoli is to a large part taken-up at the same time as carbon dioxide is given off. This occurs during inhalation. During exhalation a gas mixture is pressed out from the respiratory passages and into the tube 1, said gas mixture consisting of carbon dioxide, a certain amount of moisture as well as the part of the gas mixture which is supplied through the tube 35 and which never reached the lung alveoli but remained in the respiratory passages and the oropharyngeal tube 3 which is present.

After a few breathing cycles a steady state condition is reached in which, with each inhalation, the patient is supplied both with a mixture of narcosis gas and oxygen gas which is supplied through the tube 35 as well as with the above described gas mixture which remains in the tube 1 after the previous exhalation. This comprises, as previously stated, not only the remains of the previously supplied mixture of narcosis gas and oxygen gas but also carbon dioxide and water vapour. Together these provide the patient with a far more suitably adapted mixture of gas which, apart from the portion of narcosis gas, is very similar to the gas mixture which the respiratory organs are intended to work with. The risk of hypocapnia is eliminated almost completely with the correctly adjusted amount of fresh gas. By "correct amount" is hereby to be understood that the supply of oxygen by means of the fresh gas should not exceed such an amount per unit of time that the gas mixture containing carbon dioxide, which is to be pulsed backwards and forwards in the tube 1, is suppressed to too high a degree. On the other hand the oxygen gas must be supplied in such an amount that a sufficient oxygenation of the tissues of the patient is maintained. The amount of narcosis gas in turn must of course fulfil the requirement of keeping the patient asleep which occurs by the correct proportioning with respect to the total flow of fresh gas.

The amount of inhaled gas mixture which is supplied to the patient per unit of time is dependent both on the volume in each respiratory cycle which is determined by the patient's bodily constitution and by the respiratory frequency which, with respiratory assistance, can be controlled with the help of the ventilator 7. How the respiration continues, independent of whether this is spontaneous or assisted, can be controlled by the movement of the bellows 22. An exhalation produces an overpressure in the system which propagates from the tube 1 via the valve 14 and the tube 20 to the chamber 24 so that the bellows lifts. In the same way an underpressure propagates during inhalation to the chamber 24 so that the bellows collapses. In this way the volume per respiratory cycle and the respiratory frequency can be monitored for supplying artificial breathing assistance by means of the ventilator if this is required.

If one only considers the parts of the anaesthesia apparatus described up until now the construction corresponds to a large degree with that of the Bain-system, particularly with its variant the Mapleson D-system. With such a system the surplus gas which occurs due to supplying fresh gas the whole time will be successively pressed further out through the connection tube, in this case the tube 1, in order to exit from the apparatus via an overflow valve which is represented here by the valve 27. Since the exhaled gas comprises a not inconsiderable proportion of narcosis gas, a not inconsiderable surplus of narcosis gas will remain unused and during release will have an undesirable effect on the environment. At the same time a certain leakage to the surroundings closest to the patient can also not be avoided which, due to the relatively high narcosis gas content, will worsen the working environment around the patient. Due to the necessary surplus of fresh gas there will also be a high consumption of narcosis gas and oxygen with increased gas cost as a result. These inconveniences increase with the increased requirement of the patient for narcosis gas and oxygen gas, said requirement as mentioned being related to body weight to a certain degree.

A requirement of 5 l/min. of fresh gas to a patient with a body weight of 70 kg has been measured with the Bain-system. Since one is no longer prepared to accept said disadvantages which arise due to such a high supply of fresh gas there has, to a large extent, been a switch-over to the use of the circle system with re-generation of the exhaled gas so that its remaining content of narcosis gas and even oxygen gas can be used in the continued respiration circle. In this way the consumption of gas can be reduced down to about 10% of that described, however with the mentioned risk for hyperventilation due to the highly reduced proportion of carbon dioxide in the inhaled gas.

The risk of hypocapnia is avoided in the present system as described by the return flow of exhaled gas which is collected from the tube 1. The volume in the tube 1 is however limited and at a certain volume of exhaled gas the tube 1 will be overfilled so that a surplus, which increases with increased volume of exhalation, will be passed into the tube 11 and via the valve 14 through the branch 12 and further through the absorber 18 to be sucked into the tube 1 via the branch 13 and the valve 15 at the next inhalation and in such a way can subsequently be used beneficially by the patient. The surplus which arises due to the successively supplied fresh gas and for which there is no room in the system will, as previously described, exit via the overflow valve 27.

This means that up to a certain respiratory volume the system in the anaesthesia apparatus will function essentially in the same manner as the Bain-system with re-inhalation of non carbon dioxide-free inhalation air. Above a certain respiratory volume a successively larger part of the exhaled air will, with increased volume, be regenerated in the absorber 18 in order to be re-supplied to the patient and does not need to be lost as with the Bain-system through the overflow valve. The reduced discharge means a reduced requirement of fresh gas which is that which should be achieved. Conversely the part of the apparatus which is represented by the tube 1 with its tube 35 for fresh gas supply will function according to the principal of the Bain-system and give an inhalation contribution of carbon dioxide which is required in order to avoid the risk of hypocapnia, which was also something to be achieved.

The chosen volume in the tube 1 is very important. This volume constitutes a sort of reservoir for carbon dioxide-containing gas. If the volume is too small the system will function purely as a circle system to such a large extent that the associated risk of hypocapnia will be present, whilst if the volume is too large an economical use of fresh gas, to which the work of the absorber should contribute, will be far too little.

By taking account of the bodily build and the weight of a frequently occurring patient group within the main range of 40-90 kg body weight and by practical tests it has been determined that the volume in the tube 1 should be within the region of about 300 ml. Within this range, the risk for hypocapnia is practically eliminated at the same time as the fresh gas consumption level can be kept at a value which essentially corresponds to the consumption with the circle system. Within the range the flow of fresh gas can be regulated to a certain weight amount per unit of time, which to a large degree can be normalized. In this way the specific requirements of the patient can be regulated e.g. merely by regulation of the respiratory frequency.

The system can be used outside the range of 40-90 kg body weight, whereby with lower body weight it will function to a greater degree as a pure Bain-system whilst at higher body weights it will function similarly to the circle system. If it is deemed desirable the apparatus can be adapted to work as a hybrid between the Bain-system and the circle system as within the said range by adaptation of the volume in the tube 1. This can be particularly useful where larger volumes per unit of time are required. For patients outside the range to which the apparatus should be adapted, a regulation of the fresh gas supply per unit of time must be effected and determined according to the prevailing conditions.

The system has been tested practically and the fresh gas supply as well as the re-inhalation of carbon dioxide has thereby been recorded, the latter having also been registered through examination of the blood content during narcosis (amount of FeCo₂). This has thus shown that an apparatus built according to the invention gives a gas saving which approaches an apparatus built according to the circle system and a carbon dioxide take-up, which corresponds with normal values.

## Claims

1. Anaesthesia apparatus comprising a number of flow channels with connection means (2, 3) for connection to a patient's respiratory passages, means (34) for connection to a gas source (40) for supplying narcosis gas and oxygen gas to the apparatus and preferably also a unit (6) for control of the respiratory process by registering the pressure variations in the flow channels and preferably also at least one unit (7) for assisting respiration of the patient by producing rhythmic pressure changes in the flow channels, **characterized by** the combination of an outer conduit (1) forming one of said flow channels which, at an outer end, is connected to said connection means (2, 3) to the patient and, with an inner end (4), is connected to a second of the flow channels of the apparatus and via these to the preferably arranged units for respiratory control (6) and respiratory assistance (7), a gas conduit (35) preferably placed in the outer conduit (1), said conduit (35) at one end being connected inside the outer conduit alongside the patient connection (2, 3) and which, at its other end, is connected to the gas source (40), at an inner end (4) to the outer conduit (1) a connection leads this to additional ones of said flow channels (5, 11, 12) for circulation which is arranged by input gas through the conduit (1) into a circulation system which comprises an absorber (18) for carbon dioxide in said gas, with valves (14, 15) arranged to direct the gas circulation in such direction that gas supplied from the outer conduit (1) is supplied to the absorber (18) through the same and out of the same as well as back into the outer conduit (1) whereby the outer conduit (1), between its patient connection (2, 3) and its connection to the circulation channels (12, 13, 18), is arranged to enclose such a volume of gas that a reservoir of exhaled gas from the patient is formed, from which, during inhalation, gas is collected together with the gas from the gas source (40) and supplied through the conduit (35), the size of which reservoir is adapted to give patients inside a predetermined range of required inhalation gas such a contribution of such gas during each respiratory cycle quantified with carbon dioxide and formed during exhalation, that the risk of hypocapnia by a relatively too large contribution of oxygen gas can be controlled at the same time as exhaled gas, for which there is no space in the reservoir, passes into the flow channels for circulation through the absorber (18) and returns to the reservoir formed by the conduit (1) for re-supply to the patient, whereby the content of narcosis gas and oxygen gas in said surplus gas can be put to beneficial use.

2. Anaesthesia apparatus, **characterized** **in** that the outer conduit (1) is so arranged that it forms a reservoir volume of substantially 300 ml, whereby the apparatus is advantageously adapted with respect to the risk of hypocapnia and gas consumption, for the patient group within the body weight range of 40-90 kg.
